# EUROPEAN PATENT APPLICATION

(11) **EP 0 641 555 A1**
(43) Date of publication of application: **08.03.1995**
(21) Application number: 94300285.7
(22) Date of filing: 17.01.1994
(51) Int. Cl.: A61H 23/00

(54) **Hand held massager with rotating head**

(30) Priority: 03.09.1993 US 116986
(71) Applicant: SUNBEAM CORPORATION, Fort Lauderdale, Florida 33301-2100 (US)
(72) Inventor: Burroughs, Andrew Chritopher, Wisconsin 53143, (US); Lake, John Andrew, Evanston, Illinois 60202 (US); Sampson, Craig Field, Illinois 60044 (US)
(74) Representative: Howden, Christopher Andrew

(57) **Abstract**

A vibrating, rotating massager (1). The massager (1) includes a vibration means (20) mounted to a fixed axle (16) about which a spherical massager head (30) rotates. The vibration of the massage head (30) is isolated from the handle (10) of the massager (1) by elastomeric bushings (14).

## Description

### FIELD OF THE INVENTION

The present invention relates to a motorized, hand-held massaging apparatus having a rotating massage head. In order to maximize user comfort, the invention reduces the vibrations that are felt through the handle of the apparatus, on massagers having either rotating and non-rotating massage heads.

### DISCUSSION OF THE PRIOR ART

It is known in the art to construct non-motorized, hand-held massagers with rotating massage heads. See United States Patents No. 2,213,356 (S. D. Dunlap), 4,622,956 (Nesheim), 4,989,585 (Auker), Des. 182,123 (Kaliski), Des. 276,652 (O'Brien et al.), Des. 285,724 (Grundler et al.), and Des. 293,471 (Sato). These references disclose devices having various types of rotating massage heads, such as those having one or more rotatable or non-rotatable balls mounted on a shaft, a rotatable hub with a plurality of teeth located on the periphery of the hub and extending from the hub in a radial direction, and an ellipsoid with raised longitudinal fingers extending from end to end of the ellipsoid.

It is also known to construct massagers containing motors. These motors are used to create vibratory or other motions in order to aid in the massage. Some massagers provide a massage from a non-rotating massage head. See United States Patent No. 4,722,326 (Ruderian). These devices may generate friction and discomfort when moved across the skin because the massage head must be dragged across the skin instead of rolled over it. Also, the constant vibration, at the same frequency, tends to produce an undesirable numbing effect on the hand holding the device.

Other massagers provide a motorized massage with a rotating massage head in order to eliminate the need for lotions or oils to reduce friction. See United States Patents No. 1,958,936 (R. Bajette et al.), 2,988,084 (W. A. Douglas), 3,845,758 (Anderson), 3,994,290 (Springer et al.), 4116,233 (Scaduto), 4,565,189 (Mabuchi), and 5,123,406 (Masuda). These devices generally comprise massagers with the motor located either in the handle, or in a massage head which is rigidly attached to the handle. This design also subjects the holder of such a device to continuous vibrations which may cause fatigue and discomfort. It is known in the art to use coil springs as a means of isolating the massage head from the handle.

The art teaches the use of a heating element in a massager. See United States Patent No. 2,809,630 (W. Volker). The art, however, places the heating element within the massage head but not in contact with it. This design of a heating element is inefficient because the air between the heating element and the massage head acts as an insulator and increases the time required for the massage head to heat up.

### SUMMARY OF THE INVENTION

The present invention relates to a motorized, hand-held massager with a rotating massage head. The massage head rotates about an axis that is fixed to the handle of the massager. In order to provide the most comfort to the user, the present invention minimizes the amount of vibration that is transmitted through the handle of the device, while still providing a vibratory massage to the applied surface. In order to minimize the vibration in the handle, the present invention places the motor, the source of the vibration, in the massage head and then isolates the handle from the massage head. This isolation is accomplished through the use of resilient bushings to attach the massage head to the handle of the device. The combination of resilient bushings and placing the motor within the massage head permit the massage head to vibrate somewhat independently of the handle, thus reducing the amount of vibration transmitted through the handle and to the hand of the user holding the device. The present invention uses a rigid massage head so that the vibratory massage force, while partially isolated from the handle, is transmitted to the applied surface and not absorbed, as would be the case if the massage head were fabricated of a soft material. In addition to providing vibrational isolation, the resilient bushings provide a resilient feel when applying loads to the massage surface.

The massage action is generated by a vibration means within the massage head itself. The position of the vibration means is fixed with respect to the handle of the device and does not rotate with the massage head. In a preferred embodiment, the vibration means is fixed to the axle about which the massage head rotates. The vibrational motion can be generated with either a solenoid energized by a repetitive signal, or with an electric motor driving one or more eccentric weights.

In another preferred embodiment of the invention, the frequency of vibration is automatically and periodically or continually varied to reduce fatigue to the user.

A preferred embodiment of the invention provides a heating element in the apparatus.

### BRIEF DESCRIPTION OF THE DRAWINGS

In the accompanying drawings, the preferred embodiments of the invention and preferred methods of practicing the invention are illustrated in which:

FIG. 1 and 1a show isometric views of a massager of the invention, with a portion of the housing removed, showing the internal construction of one preferred embodiment of the present invention.

FIG. 2 is an isometric, partial breakaway view of a preferred embodiment of the massage head containing a vibrator means consisting of an eccentric weight electric motor.

FIG. 2a is an isometric view of a vibration means containing a fan means integrated into the arm attaching the eccentric weight to the vibration means.

FIG. 3a is an isometric view of a preferred embodiment of the invention consisting of a counter-rotating eccentric weight electric motor having a bevel gear.

FIG. 3b is an exploded isometric view of the preferred embodiment shown in Fig. 3a.

FIG. 3c is a series of schematic diagrams illustrating the forces generated by the counter-rotating eccentric weight illustrated in Figs. 3a and 3b.

FIG. 4 is a schematic isometric breakaway view of a preferred embodiment of the massager containing a vibrator means consisting of a solenoid.

FIG. 5 is a schematic drawing of a heating device for one embodiment of the invention.

FIG. 6 a-b are isometric views, inside and outside, respectively, of half of a spherical massage head of the invention.

FIG. 7 is an isometric view of the elastomeric bushing and backup ring of the present invention.

FIG. 7a is an isometric view of a backup ring used to support the elastomeric bushing of Fig. 7.

FIG. 8 is an isometric view of the motor mount housing of the present invention.

FIGS. 9 a-c are isometric views of the elastomeric bushing of the present invention.

FIG. 10 is a schematic illustration of a motor speed control circuit with the potentiometer for use in a preferred embodiment of the invention.

FIG. 11 is a schematic illustration of a motor speed control circuit with discrete speeds for use in a preferred embodiment of the invention.

FIG. 12 is a schematic illustration of a motor speed control circuit and a battery recharging means for use in a preferred embodiment of the invention.

FIG. 13 is a schematic illustration of a solenoid vibrator means with frequency control for use in a preferred embodiment of the invention.

FIG. 14 is a schematic illustration of a capacitive potential divider circuit for use in a preferred embodiment of the invention.

### DETAILED DESCRIPTION OF THE INVENTION

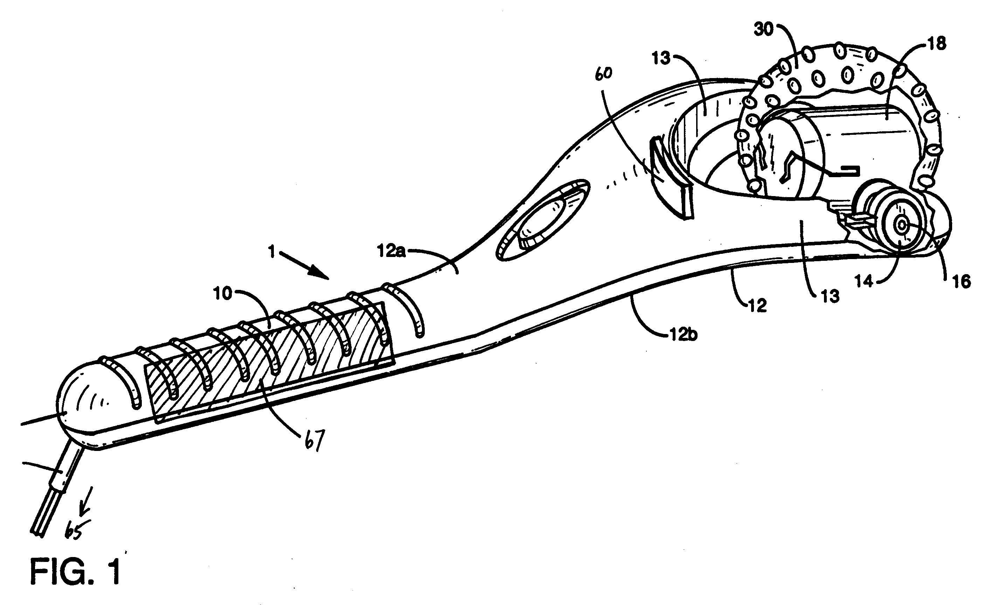

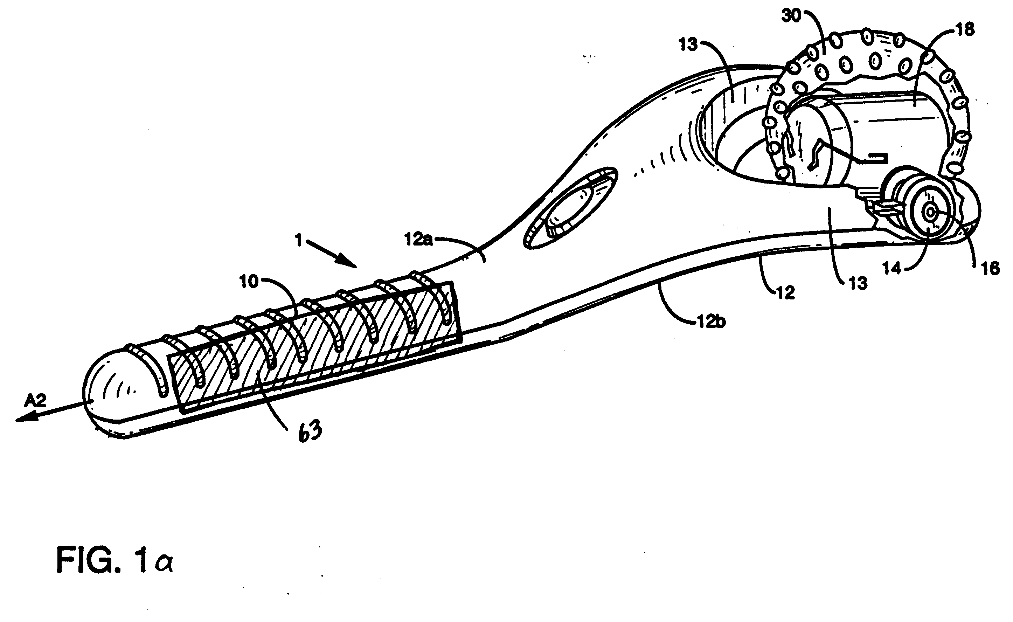

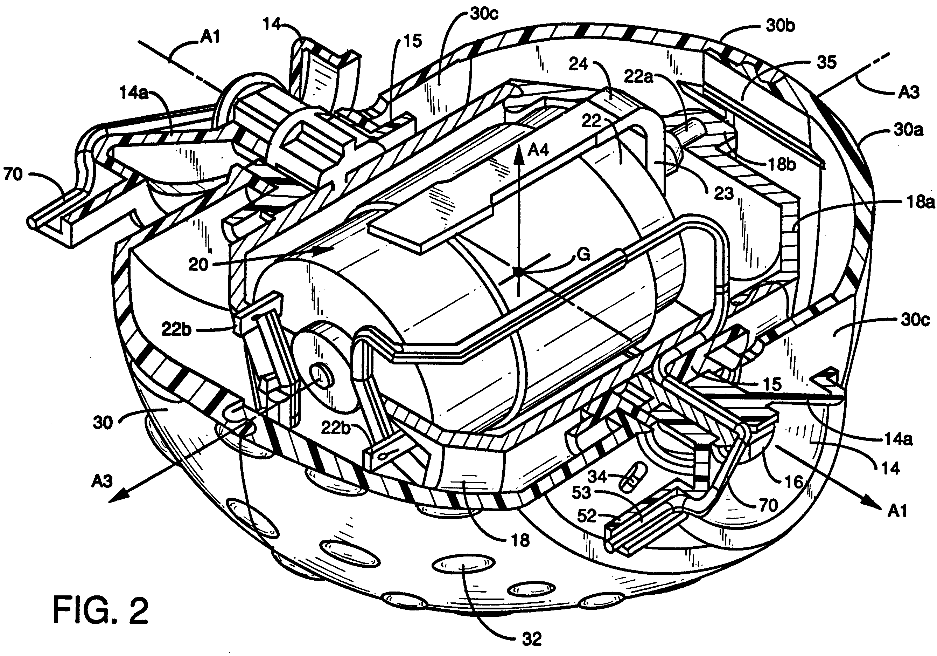

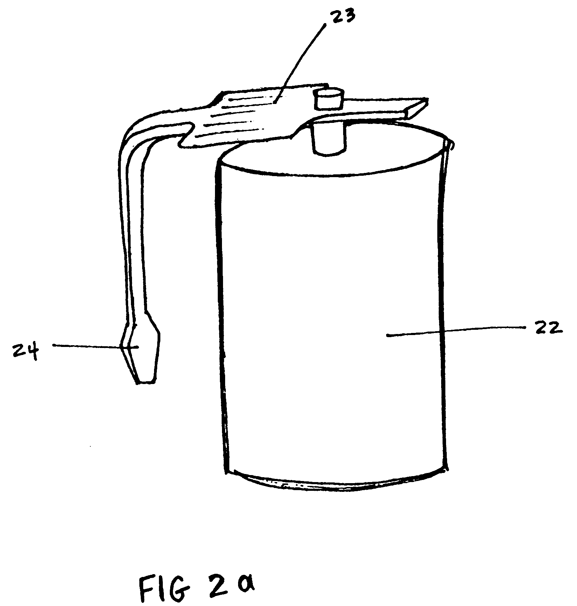

Figs. 1 and 2 show a preferred embodiment of the present invention. The massager, generally 1, comprises a handle 10, which extends into a massage head housing 12, and a massage head 30. The massage head 30 preferably rotates within the massage head housing, 12, which preferably comprises a u-shaped saddle having a pair of arms, or axle-supporting members 13. Two elastomeric bushings 14 are located in the massage head housing 12, one in each axle-supporting member 13, on opposite sides of the massage head 30. Resilient bearings 15, preferably self-lubricating, e.g. with impregnated lubricant, are positioned between the massage head 30 and the axle 16 to reduce rolling friction and to fill in any gaps that could occur due to production variances, potentially resulting in rattling of the vibrating massage head 30.

An axle 16 is fitted within each of the elastomeric bushings 14. Both the elastomeric bushings 14 and the axle 16 are preferably fixed with respect to the massage head housing 12, and do not rotate with the massage head 30. Rather, the massager head 30 rotates about the axle 16, preferably on the resilient bearings 15.

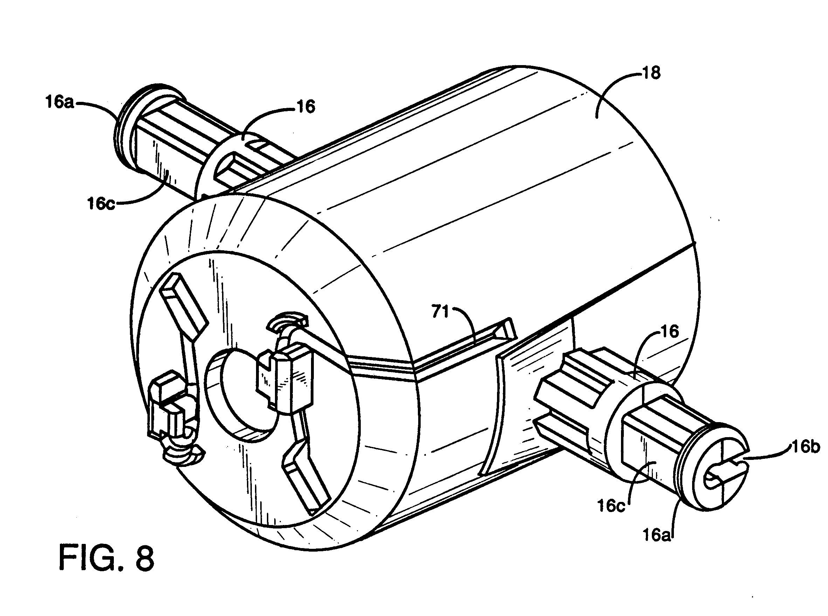

The axle 16 illustrated in Figures 1 and 2 comprises two sections, each of which is attached at its outer end to an elastomeric bushing 14, and at its inner end to a vibration means mounting bracket 18. As best seen in Figure 8, an annular flange 16a on the outer end of each axle 16 retains the elastomeric bushing 14. The axle 16 could, of course, comprise a unitary piece passing through or around the mounting bracket 18. Attached to the axle 16 and located within the massage head 30, is the vibration means mounting bracket 18, to which a vibration means, generally 20, is mounted. As best seen in Figure 2, the vibration means 20 may comprise an electric motor 22, having an eccentric weight 24. This motor 22 is preferably mounted within the motor mount 18 by mounting the centerline A3 of the motor 22 along (but not necessarily parallel to) the axis A2 of the handle, or parallel to the skin surface of the user of the device. The motor mount 18 preferably comprises a shell that allows the wrap-around eccentric weight 24 to rotate freely within the motor mount 18.

The motor mount 18 may include a shell cap 18a that is press fitted into the cylindrical motor mount 18. This shell cap 18a preferably includes a bearing 18b for rotatably supporting the shaft 22a of the motor 22. This bearing 18b may include ball bearings or a bronze bushing or other support structure known in the art. The motor mount 18 may be fabricated of any durable material, including by way of example, but not limitation, polycarbonate.

Since the vibration means 20, via the vibration means mounting bracket 18, is attached to the axles 16, the vibration means 20 is fixed with respect to the massage head housing 12 and therefore, does not rotate with the massage head 30. In the same way, the massage head 30 is free to rotate about the resilient bearings 15, stationary axles 16, mounting bracket 18, and vibration means 20, without causing rotation of the vibration means 20, vibration means mounting bracket 18, resilient bearings 15, or axles 16. If the vibration means 20 rotated with the massage head 30, wires 70 powering the vibration means would become twisted and would eventually break. Additionally, since the vibration means 20 is fixed with respect to the handle 10, it is possible to create a vibrational motion along a single axis or plane with respect to the handle 10, which does not vary as the massage head 30 is rotated. In a preferred embodiment of the invention, the massage action is directed primarily non-parallel to the longitudinal axis of the handle; that is, the massage action is directed at angles ranging from about 45° to perpendicular with respect to the longitudinal axis A2 of the handle. Most preferably, this vibrational motion is directed primarily perpendicular to axis A1 of the axle 16, and normal to the skin surface of the user of the device.

As further illustrated in Figure 1, the housing 12 preferably comprises two halves, an upper housing 12a and a lower housing 12b, which may be press fitted or screwed together. The housing 12 is preferably injection molded plastic.

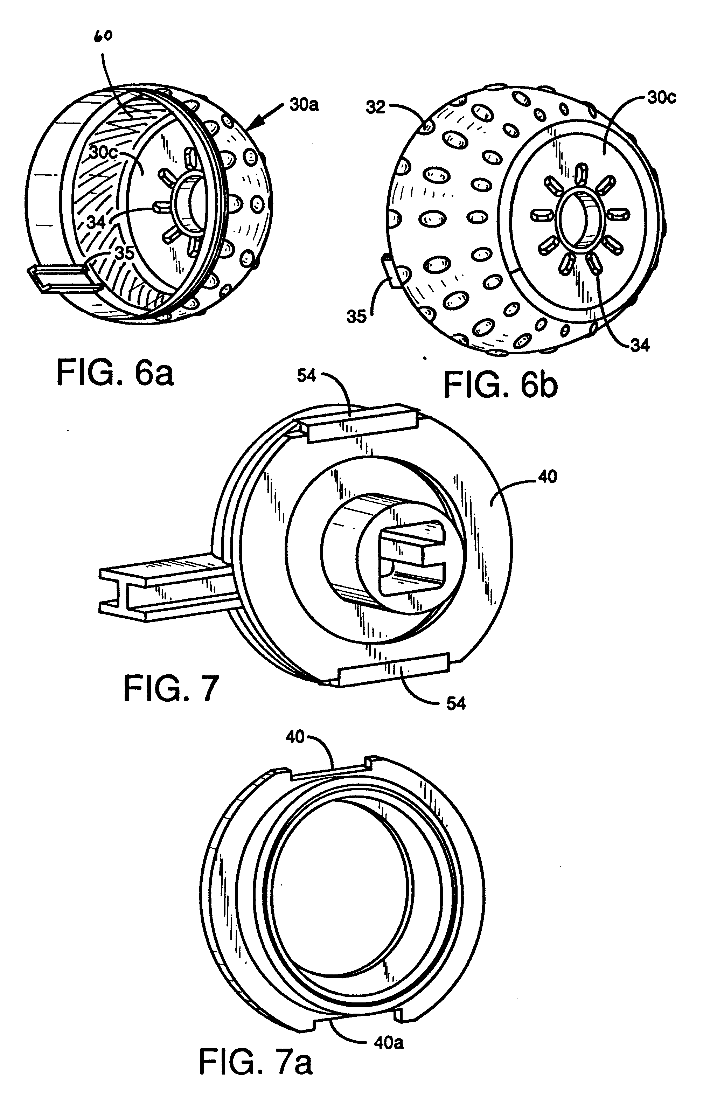

Referring now to Figures 2, 6a, and 6b, the rotary massage head 30 preferably comprises two rigid halves, a male half, 30a, and a female half, 30b, that snap together around the axle 16 and motor mount 18. Preferably, the massage head 30 has a generally spherical shape, but may have a pair of flattened ends 30c for receiving the bushings 14 and axles 16. The plastic halves of the message head 30 are preferably fabricated of injection molded plastic, such as ABS, including for example, CYCOLAC GSM Resin, manufactured by General Electric. The massage head 30 may include a plurality of protuberances or indentations 32 on the outer surface as illustrated, for increasing the massage action of the device. One section of the rotating massage head 30 may further include an alignment flange 35 for aligning the two halves during fabrication. The rotating massage head 30 may also include means for preventing rotation thereof, such as a stop (not shown) for fixedly engaging the massage head 30 to the axles 16.

The vibration means 20 is in direct contact with the axles 16 through the vibration means mounting bracket 18 so that the vibrations generated by the vibration means 20 are transmitted directly to the massage head 30 through the resilient bearings 15 and axles 16 upon which the massage head 30 is rotatably mounted. These vibrations, however, are isolated from the massage head housing 12 and the handle 10 by the elastomeric bushings 14, which allow the axles 16 to oscillate with minimal transmission of that oscillation to the massage head housing 12 or the handle 10. The ends of the axles 16 are preferably keyed within the elastomeric bushings 14 or otherwise secured therein so that the axles 16 do not rotate.

As previously discussed, Fig. 2 shows a preferred embodiment of the massage head 30 in which the vibration means 20 consists of an electric motor 22, and a "wrap-around" eccentric weight 24 which is attached to the electric motor 22 through an arm 23. As the "wrap around" eccentric weight 24 rotates around the electric motor 22, the massage head 30 vibrates on a plane normal to the axis of rotation A3 of the eccentric weight 24. This plane generally passes through axes A1 and A4. The amount of movement of the massage head 30 is affected by the mass of the rotating eccentric weight 24, the mass of the assembled components inside the massage head 30, and the stiffness of the elastomeric bushings 14.

The shape of the elastomeric bushings 14 controls their relative stiffness in a particular direction, and thus the path of movement of the massage head 30 on a plane normal to the axis of rotation A3 of the eccentric weight 24 can be manipulated by changing the bushing's shape. In a preferred embodiment of the invention, the relative stiffness of the elastomeric bushing 14 is approximately equal in the three axes A1, the axis of rotation A3 of the eccentric weight 24 and a third axis A4 perpendicular to the preceding two. The axis of rotation A3 of the eccentric weight 24 does not necessarily coincide with the axis of the handle A2. They may be inclined to each other, and in fact, they are, by as much as 45 degrees in a preferred embodiment of the invention.

It has been found that when using a wrap-around eccentric motor 22 in the massage head 30 the best results are achieved when the net center of gravity "G" of the vibration means, e.g., motor 22, the arm 23, and the weight 24, i.e., the center of gravity "G" when the eccentric weight is spinning, is on the axis A1 defined by the axles 16. Most preferably, the net center of gravity of the vibration means is at the midpoint of the axle 16 provided that the device is symmetrical about an axis A2 of the handle. Most important, the net center of gravity of the eccentric portion alone of the rotating wrap-around weight should lie on or close to the intersection of axis A1 and axis A3. Most preferably A1 and A3 should intersect. When the net center of gravity of the combined motor 22, arm 23 and weight 24 is not located on the midpoint of the axis A1, the vibration generated is inefficient, including a component of oscillating rotational motion of the motor 22, motor mount 18, and wrap-around eccentric weight 24 about the axis A1. This motion is undesirable because it causes energy to be expended without being able to transmit this motion to the skin of the user; since the massage head 30 is free to rotate about the axis A1, any moments generated about this axis A1 by the eccentric motor 22 cannot be transmitted through the resilient bearings 15 to the massage head 30 as the resilient bearings 15 will not transmit torque about their axis of rotation A1. The energy that is expended in creating this oscillating motion is not available to generate the more desirable movements of the massage head 30 and so the effectiveness of the massage is diminished.

It is also preferred that the motor arm 23, and weight 24 spin about an axis A3 which is perpendicular to the axle 16 axis A1, and substantially parallel to the skin surface of the user of the device. The motor arm 23 and weight 24 may comprise a unitary piece or may be two separate pieces fastened together.

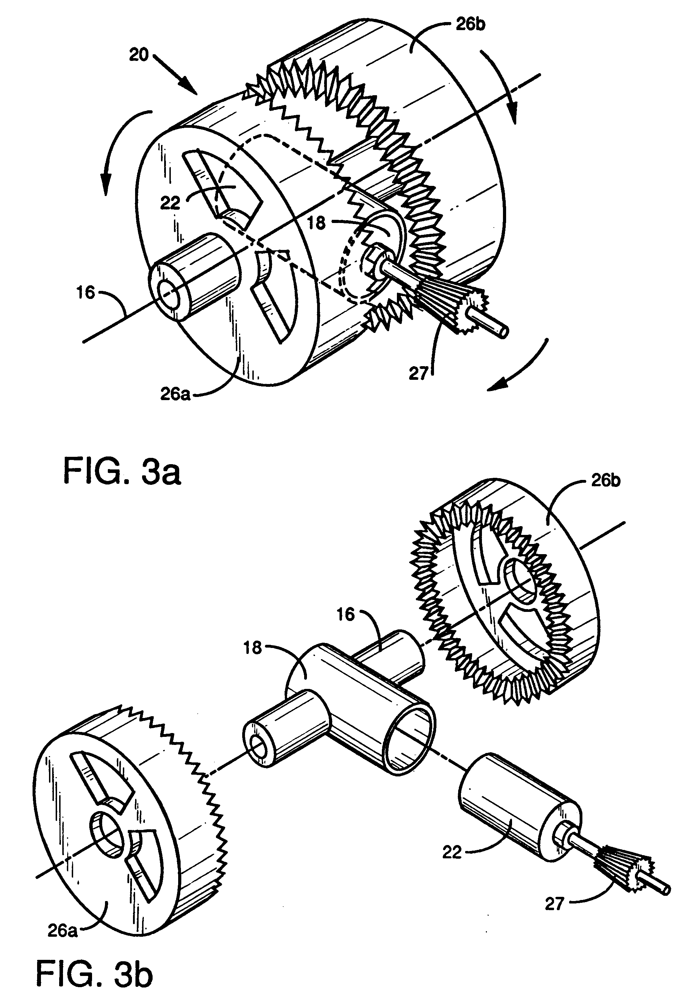

Figs. 3a and 3b show another preferred embodiment of the vibration means 20. FIG. 3a shows the vibration means 20 as it would be assembled in the massage head 30. FIG 3b shows an exploded view of the vibration means 20. This vibration means 20 consists of an electric motor 22 with a pair of counter-rotating eccentric weights 26a and 26b. These weights 26a and 26b are mounted on and rotate about the axles 16. Therefore, the axles 16 act as the axis of rotation of the weights 26a and 26b. The counter-rotating weights 26a and 26b comprise eccentric bevel gears and are connected to the electric motor 22 through, and driven by, a bevel gear pinion 27.

The embodiment of Figs. 3a-c includes a motor mount 18 having a pair of gear axles 16 extending therefrom. Preferably, the eccentric bevel gears 26a, 26b and the massage head 30 all rotate about these stationary axles 16. The axles 16 may be received by the flexible isolation bushings 14 as previously described.

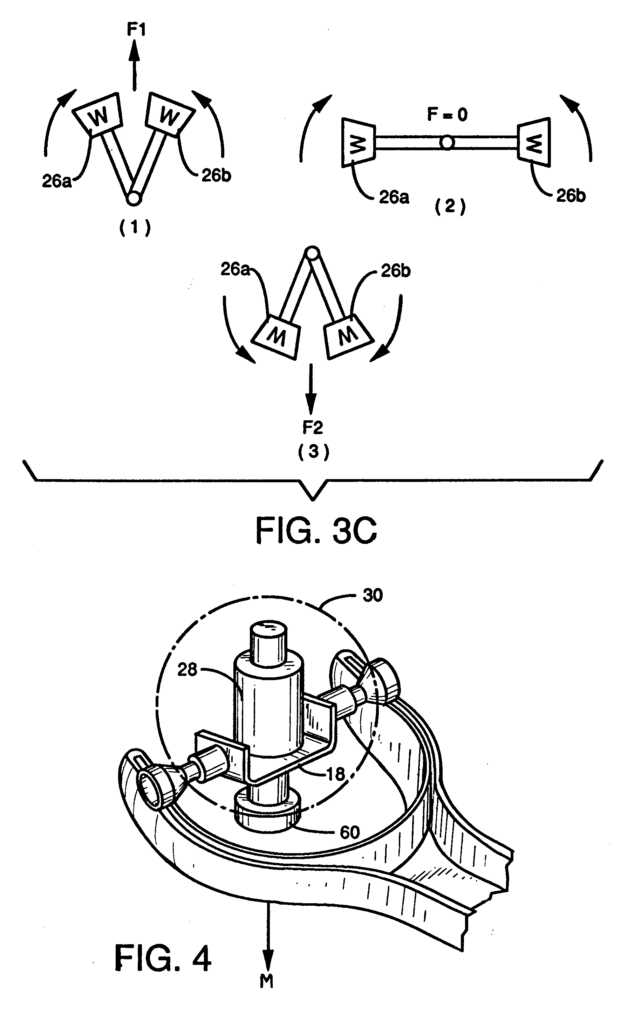

Fig. 3c shows the forces generated by this vibration means. As shown in Figs. 3c(1) and 3c(2), vibrational forces are maximized when the counter-rotating weights 26a and 26b come together and pass by each other. When this occurs, a force F1 in Fig. 3c(1), F2 in Fig. 3c(3), is generated in a radial direction from the axis of rotation of the weights 26a and 26b towards the point where the weights 26a and 26b pass each other. When the weights 26a and 26b are opposite each other as illustrated in Fig. 3c(2) no net force is generated. As a result, this embodiment of the invention provides vibration primarily in one direction, i.e., normal to the user's skin surface, with respect to the massage head housing 12 and the handle 10.

Fig. 4 shows another preferred embodiment of the massage head 30 which also generates a vibratory motion along a single axis. The vibration means 20 of this embodiment consists of a solenoid 28 energized by a repetitive signal that drives a mass 60 within the rotating massager head 30 in the direction of "M". The solenoid 28 and mass 60 are mounted within a mounting bracket 18 substantially as described herein with respect to rotating motors. In this embodiment, virtually all components of vibrational motion may be directed normal to the user's skin surface, i.e., in the direction "M".

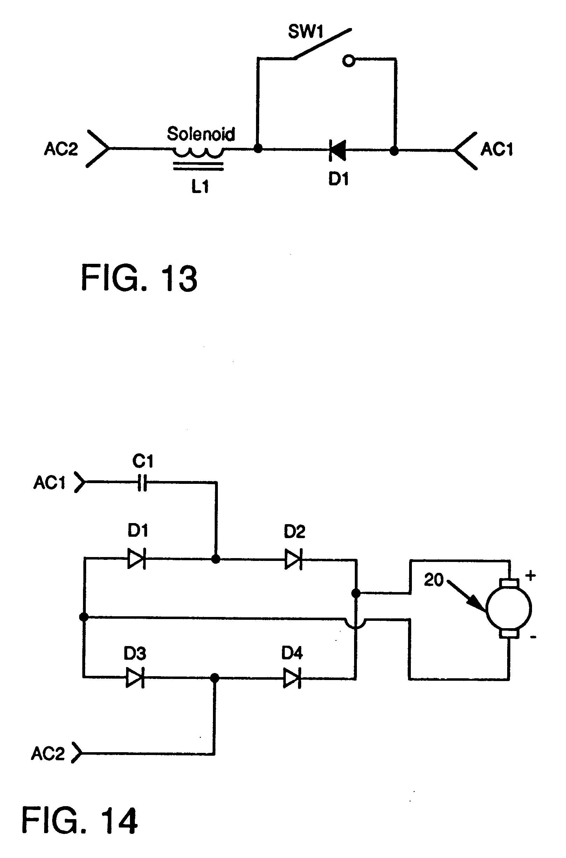

Figure 13 shows a schematic drawing of a solenoid vibration device including a means for varying the frequency. The solenoid L1 includes a spring means (not shown) so that when the solenoid L1 is not energized it is always returned to the same state. When switch SW1 is closed the diode D1 is bypassed and current flows through the solenoid L1 in both directions as the source AC1 and AC2 provides alternating current. As a result the solenoid L1 will produce a vibration every time it is energized or at a rate of twice the line frequency, corresponding to a high frequency for the massager. When switch SW1 is open, diode D1 restricts current flow to only one direction. When the appropriate half-cycle energizes the solenoid L1 a vibration is produced. During the other half-cycle when the diode D1 prevents current flow, the spring returns the solenoid L1 to its previous state producing little or no appreciable vibration. As a result, the solenoid L1 produces a vibration only once per cycle or at a rate equal to the line frequency, corresponding to a low frequency for the massager.

If an electric motor is exposed to excessive heat, it will fail prematurely. In order to dissipate the heat generated by the electric motor, the preferred embodiment of the invention contains air vents 34 in the massage head 30. These vents 34 are preferably placed in a less visible part of the massager head 30, such as the flat portion 30c near the bushings 14, and allow the heat generated by the electric motor 22 to escape from the massage head 30. To provide additional cooling, the electric motor 22 shown in Fig. 2 may contain a fan blade 23 integrated into the arm 23 connecting the wrap around eccentric weight 24 to the electric motor 22 as shown in Fig. 2a. This fan blade 23 rotates with the eccentric weight 24 when the motor 22 is energized and circulates air within the massage head 30, moving hot air away from the electric motor 22. This hot air will eventually escape through the vents 34 in the massage head 30. The mounting bracket 18 also preferably has openings for inletting and exhausting air for cooling the motor by providing for a continuous flow of air through the bracket 18.

Every object has a natural frequency of vibration. This is the frequency at which the object can most easily be made to vibrate. Experiments have shown that the handle 10 in the present invention vibrates an acceptably small amount when the vibration means 20 is vibrating at least at 150% of the natural vibration frequency of the apparatus. Thus, a preferred embodiment of the present invention sets the lowest vibration speed of the vibration means 20 at approximately 150% of the natural frequency of the massager, or about 5000 Hz.

Additionally, it has been determined that vibration means 20 that generate vibrations which are perpendicular to the longitudinal axis A2 of the handle 10 are superior, for massaging purposes, to vibrations which are parallel to the longitudinal axis A2 of the handle 10. This is because a person using a hand-held massager tends to hold it at a point along the handle which is approximately the center of gravity for the entire unit.

When the massage head generates vibrations which are perpendicular to the longitudinal axis A2 of the handle 10, the entire unit tends to move in a manner that can be characterized as a repetitive, partial-rotation of the unit about the center of gravity of the unit. At the extremities of the unit, the resulting motion is the most severe. In contrast, the effects of this motion are minimized at the center of gravity of the unit and result in only a slight, repetitive, partial-rotational motion there. This location is known as the "center of percussion". Thus, the user's hand does not feel the full effect of the transmitted vibrations at the massage head 30 which are perpendicular to the longitudinal axis A2 of the handle 10.

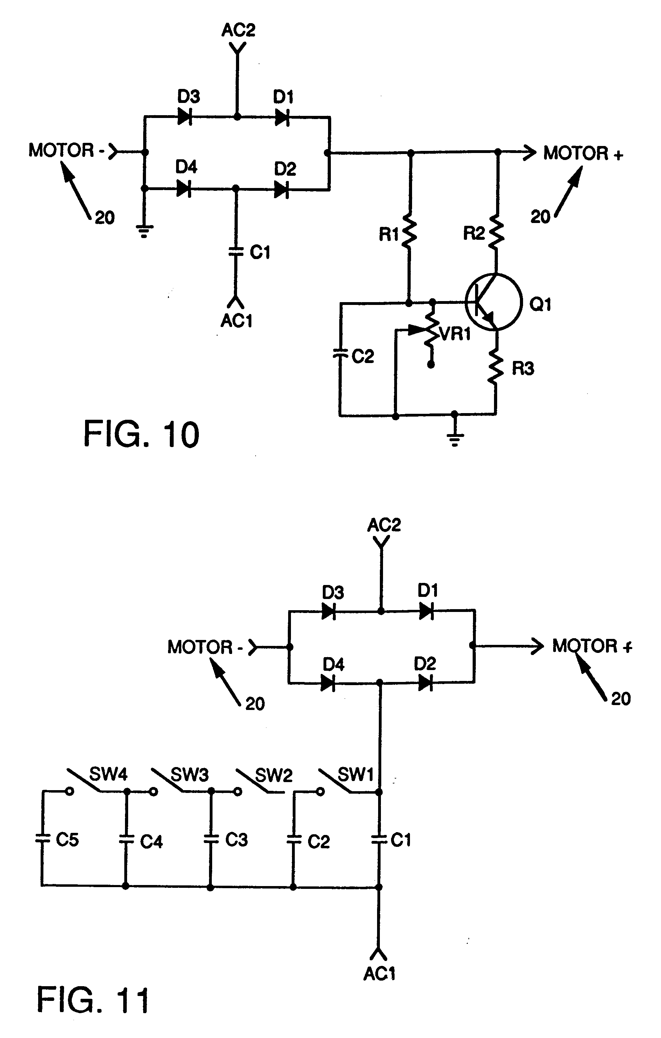

On the other hand, when the unit generates vibrations which are parallel to the longitudinal axis A2 of the handle 10, the motion transmitted to the handle 10 causes it to move linearly, not rotationally. There is no position on the handle where the linear vibratory motion transmitted to the user's hand is reduced, and therefore, discomfort and/or numbness to the user's hand may result, especially when isolation bushings 14 are not employed. In another highly preferred embodiment of the invention, the vibrator speed may be varied, either smoothly or at discrete frequencies. In one embodiment, the vibrator is varied using a slide-type switch that governs a rheostat for varying the frequency infinitely within upper and lower limits. Figure 10 is a schematic drawing which illustrates one embodiment of the present invention which uses a rheostat to smoothly vary vibrator speed. The diode bridge D1, D2, D3 and D4 produces only positive voltage across the vibration means 20. The average DC voltage across the vibration means 20 is determined by the impedance of the vibration means 20 and the amount of current provided to it. By reducing the current going to the vibration means 20, the average DC voltage across the vibration means 20 will be reduced, and as a result the frequency of the vibration means 20 will also be reduced. In this circuit, the current to the vibration means 20 is reduced by diverting some of the current through resistors R2 and R3 and transistor Q1. The amount of the current diverted through these components is determined by the magnitude of the base current in Q1. The magnitude of the base current can be changed by adjusting the variable resistor VR1. A smooth variation in vibration frequency is achieved by adjusting the variable resistor VR1.

Figure 11 shows the schematic drawing which illustrates a means for providing discrete and finite frequency variations in the vibration means 20. The capacitors C2, C3, C4 and C5 can be connected in parallel with the capacitor C1 by closing the appropriate switches SW1, SW2, SW3 and SW4. By increasing the current going to the vibration means 20, the average DC voltage across the vibration means 20 will be increased, and as a result the speed of the vibration means 20 will also be increased. In this circuit, the current to the vibration means 20 is increased by increasing the magnitude of the capacitance in series with the AC voltage source AC1. In contrast, when a switch, SW1, SW2, SW3 or SW4, is opened, it removes series capacitance from the AC voltage source AC1 and the current to the vibration means 20 is reduced, which in turn reduces the frequency of the vibration means 20. In another embodiment, a detent-engaging switch provides discrete and finite frequency variations. Other means for varying vibrator frequency are also possible as will now be readily apparent to those of ordinary skill in the art.

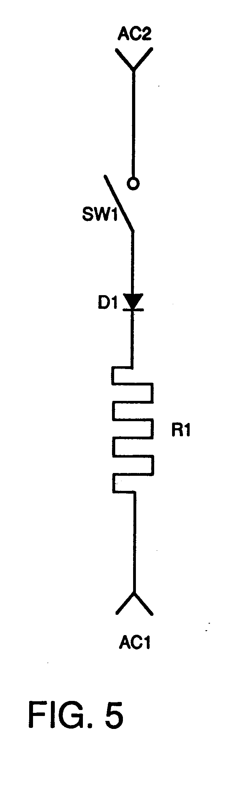

As will now be readily appreciated by those of ordinary skill in the art, the massager 1 may also include a heating element 60, of conventional design, which may be placed inside the rotating massager head 30, as shown in Fig. 6a, or within the handle 12 near the massage head 30, as shown in Fig. 1. The heating element may be powered by the same power source as the vibration means 20, and may comprise a resistive element, positive temperature coefficient element, or infrared element. Figure 5 shows a schematic drawing of a heating means 60 using resistive element R1. The switch SW1 allows the heating means 60 to be operative only when desired by opening or closing the switch SW1 accordingly. Additionally, the diode D1 half-rectifies the AC current allowing it to flow through the heating element R1 for only one-half of each cycle. This reduces the total amount of current flowing through the heating element R1 and reduces the amount of resistive material required to achieve the desired heat output. Optionally, the heating element may itself comprise the vibration means 20, which, especially in the case of an electric motor 22, will heat up during use. The degree of heating may be adjusted by varying the motor cooling device, for example, by opening or closing air vents 34 in the massage head 30, or by varying the speed of the motor cooling fan 23.

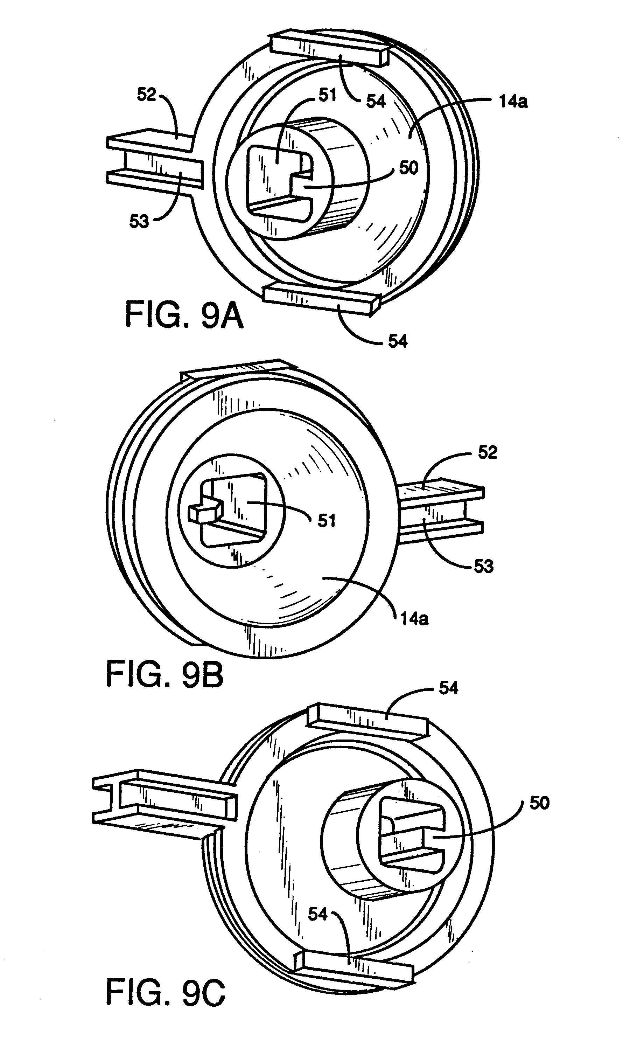

Figs. 9a-c show isometric views of the elastomeric bushings 14 used in the preferred embodiment. The portion of the bushing 14 that attaches to the axle 16 is preferably keyed 50 to accept a groove 16b in the axles 16 and prevent the axles 16 from rotating with respect to the handle 10 and the housing 12. As best seen in Figure 8, each of the axles 16 preferably has a square profile portion 16c for receiving a complimentary square hole 51 in the elastomeric bushing 14, as illustrated in Figures 9a-c.

It has been found that the most effective isolation is achieved with a bushing 14 that has approximately equal axial and radial flex. Axial flex is flex by the bushing 14 in a direction parallel to the axles 16. Radial flex is flex by the bushing 14 in a direction perpendicular to the axles 16. This approximately equal radial and axial flex is achieved by means of a bushing 14 comprising a tubular or conical section 14a as illustrated in Figures 2 and 9a-c. Flex can be increased in the axial direction by allowing this conical section 14a to approach the shape of a flat disc, whereas radial flex can be increased by allowing the conical section 14a to approach the shape of a cylinder.

The elastomeric bushings 14 preferably include a tab 52 having a slot 53 which serves to guide motor wires 70 from the handle through a hole or slot 16b in the axle 16 as illustrated in Figure 2. The wires 70 continue through the resilient bearings 15 and the motor mount 18 to the motor wire connectors 22b. The motor mount 18 may have a series of wire guide channels 71 for receiving the wires 70, as illustrated in Figure 8.

The flexible, non-rigid qualities of the bushing 14 which provide isolation also create the potential for the bushing 14 to fold or collapse on itself. In order to prevent this, a back-up ring 40 is attached to the bushing 14 to add support, as illustrated in Figure 7. The location of the back-up ring 40 prevents it from interfering with either the axial or radial flex motions of the bushing 14 and therefore no performance loss is associated with the use of the back-up ring 40.

The back-up ring 40 is preferably captured by one or more capture flanges 54, which engage a flat surface or notch 40a best seen in Figure 7a, on the back-up ring 40 and prevent it from rotating. The back-up ring 40 is preferably fabricated of any injection molded plastic such as nylon, ABS, and polypropylene.

As further illustrated in Figure 1, the elastomeric bushing 14 and back-up ring 40, which prevents the bushing 14 from collapsing on itself, are captured by the housing 12, for example, by a series of flanges (flanges) contained within the member 13 of the u-shaped saddle.

The bushing 14 is preferably fabricated of "springing" material, such as natural or synthetic rubber or a polyurethane, not a damping material such as Isoloss™, manufactured by E.A.R. Co. Alternatively, non-elastomeric spring-type materials, including by way of example, but not limitation, steel coil springs, could also be used. A springing material has a low hysteresis and tends to reflect energy without absorbing it. Damping materials have a higher hysteresis than springing materials and they tend to absorb energy. This characteristic of damping materials tends to both reduce the vibration in the massage head 30 and transmit some of the absorbed vibration to the handle 10. Both of these characteristics of a damping material are undesirable when isolating the massage head 30. In contrast, when operating at frequencies at least 150% of the natural frequency of the device, and because springing materials reflect motion without absorbing it, springing materials transmit less vibration to the handle 10, thereby providing better isolation, as well as minimizing the damping of vibrations in the massage head, allowing for a more effective vibration.

Although the present invention isolates most of the vibration from the handle 10, some vibration in the handle will inevitably exist. In order to further increase the comfort to the person holding the massager, a preferred embodiment of the present invention varies the vibration frequency of the vibration means 20. For example, the frequency may be varied smoothly in a sinusoidal manner. This variance in the vibration frequency is intended to prevent the discomfort associated with a constant-frequency vibration to the hand of the person holding the massager. This variance can be accomplished by automatically and smoothly varying the voltage to the motor 22. In the case of a vibration means 20 consisting of a solenoid 28, (Figure 4) variance in frequency can be accomplished by automatically and periodically varying the frequency of the signal energizing the solenoid. This may preferably be accomplished by use of an electronic circuit with a control allowing a number of discrete settings for different effects, or allowing gradual variation between different effects.

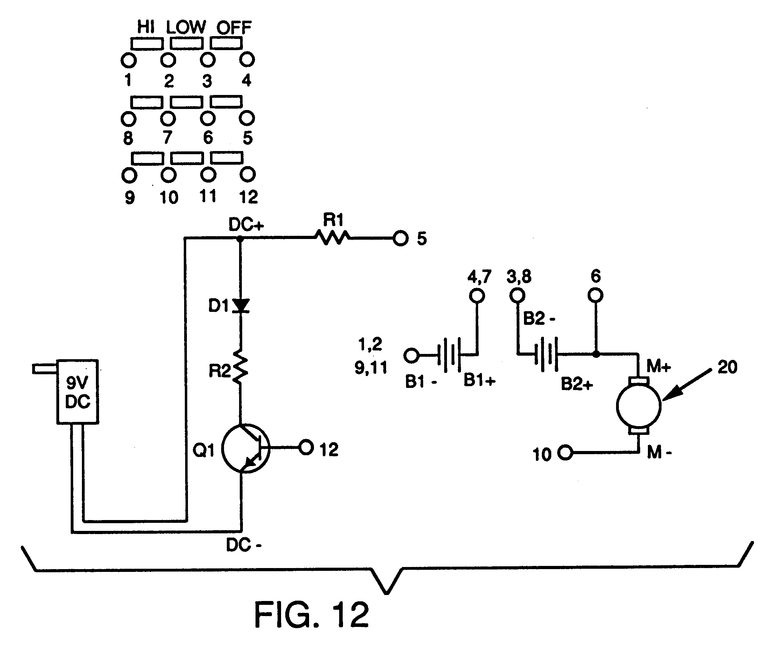

The present invention may be powered by either a self-contained, rechargeable or non-rechargeable power source 63, such as batteries, as shown in Fig. 1a. Alternatively, the present invention may use an internal power controller 67, a power cord and a fixed external power source 65, such as an AC electric outlet, as shown in Fig. 1. Figure 12 shows ciruitry for a self-contained rechargeable power source. SW1 is a 3-position, 3-pole switch. When the switch is in the 'OFF' position the batteries are disconnected from the motor circuit and are connected in series with the battery charging circuitry. When the switch is in the 'LOW' position, the two cells are connected in parallel in the DC motor circuit. In the 'HI' position, the two cells are connected in series so that the voltage to the motor is doubled and the speed is increased. The battery re-charging occurs when SW1 is in the 'OFF' position. A voltage source provides current to the batteries through R1. The charging current saturates transistor Q1 so that the light emitting diode (LED) D1 becomes illuminated. The illuminated LED is an indication to the user that the batteries are recharging.

Another preferred embodiment of the invention uses an external power source 65 with an internal switching power supply, or a capacitive potential divider, to regulate power to the vibration means 20. Figure 14 shows a capacitive potential divider circuit to regulate power to the vibration means 20. The capacitor C1 creates impeadance so that the entire voltage applied to the circuit is not applied across the vibration means 20. Figures 10 and 11 also contain capacitive potential dividers. Figure 10 uses capacitor C1. Figure 11 uses capacitors C1, C2, C3, C4 and C5. As discussed above, Figure 11 allows for discrete variable capacitance which, in turn, discretely regulates the speed of the vibration means 20. A switching power supply offers advantages over a linear power supply, including increased efficiency, resulting in less power consumption and less heat generated, and a smaller and lighter unit. Of course, particularly if space and/or cost considerations are of no moment, other power supplies known in the art could be used.

Although the invention has been described in detail in the foregoing for purposes of illustration, it is to be understood that such detail is solely for that purpose and that variations can be made therein by those of ordinary skill in the art, without departing from the spirit and scope of the invention as defined by the claims. Such variations are specifically intended to be embraced by the scope of the following claims and by all equivalents thereof.

The features disclosed in the foregoing description, in the following claims and/or in the accompanying drawings may, both separately and in any combination thereof, be material for realising the invention in diverse forms thereof.

## Claims

1. A hand-held massager (1), comprising:
(a) a handle (10);
(b) a massage head (30) disposed proximate one end of said handle (10), said massage head (30) rotatably attached to said handle (10) with stationary axle means (16) including isolation means (14) to vibrationally isolate said handle (10) from said massage head (30); and
(c) vibration means (20) located within said massage head (30) for causing a massage action through said massage head (30).

2. The hand-held massager (1) of Claim 1, wherein said isolation means (14) allows said massage head (30) to vibrate with a limited amount of oscillation in any axis with respect to said handle (10).

3. The hand-held massager (1) of Claim 1, wherein said vibration means (20) has a fixed orientation with respect to said handle (10).

4. The hand-held massager (1) of Claim 1, wherein said isolation means (14) compromises a springing material which is used to attach said handle (10) to said axle means (16).

5. The hand-held massager (1) of Claim 1, wherein said vibration means (20) is an electric motor (22) including at least one rotating eccentric weight (24).

6. The hand-held massager (1) of Claim 1, wherein said handle (10) has a longitudinal axis (A2) and said massage action is substantially perpendicular to said longitudinal axis (A2) of said handle (10).

7. The hand-held massager (1) of Claim 4, wherein said isolation means (14) comprises a generally tubular elastomeric bushing (14).

8. The hand-held massager (1) of Claim 7, wherein said massager (1) has a natural vibration frequency, and said vibration means (20) operates at a vibration frequency of approximately 150% of said natural vibration frequency of said massager (1).

9. The hand-held massager (1) of Claim 5, wherein said massage head (30) contains air vents (34).

10. The hand-held massager (1) of Claim 9, wherein said vibration means (20) further comprises an integral motor cooling means 23.

11. The hand-held massager (1) of Claim 7, wherein said tubular bushing (14) includes support means (40) to prevent said bushing (14) from collapsing on itself.

12. The hand-held massager (1) of Claim 1, wherein said vibration means (20) further includes means for automatically and periodically varying the vibration frequency of said vibration means (20).

13. The hand-held massager (1) of Claim 5, further comprising a power supply (67) selected from the group consisting of switching power supplies and capacitive potential dividers for said electric motor (22).

14. The hand-held massager (1) of Claim 5, wherein said massager (1) is powered by a rechargeable, self-contained power source (63).

15. The hand-held massager (1) of Claim 5, wherein said massager (1) is powered by a non-rechargeable, self-contained power source (63).

16. The hand-held massager (1) of Claim 13, wherein said apparatus (1) is powered by an external power source (65).

17. The hand-held massager (1) of Claim 10, wherein said motor cooling means comprises fan means.

18. The hand-held massager (1) of Claim 17, wherein said fan means is attached to said eccentric weight (24).

19. A hand-held massager (1), comprising:
(a) a handle (10) having a longitudinal axis (A2); and
(b) a rotating massage head (30) having an axis of rotation (A3), said massage head (30) being rotatably disposed with respect to said handle (10), said axis of rotation (A3) being generally perpendicular to said longitudinal axis (A2) of said handle (10); and
(c) vibration means (20) located within said massage head (30) for causing a massage action through said massage head (30), said vibration means (20) having a generally fixed orientation with respect to said axis of rotation (A3).

20. The hand-held massager (1) of claim 19, said massage head (30) being attached to said handle (10) via isolation means (14) for isolating said handle (10) from vibration of said massage head (30).

21. The hand-held massager (1) of Claim 20, wherein said vibration means (20) comprises a motor (22) fixedly mounted to an axle means (16) about which said rotating massage head (30) rotates, said axle means (16) being supported by said handle (10).

22. The hand-held massager (1) of Claim 21, wherein said handle (10) includes a substantially u-shaped saddle (13) for supporting said axle means (16), and wherein said massage head (30) rotates about said axle means (16) and said axle means (16) is non-rotatably fixedly supported by said u-shaped saddle (13).

23. The hand-held massager (1) of Claim 22, wherein said axle means (16) includes two outer ends, each axle means (16) outer end being positioned within an elastomeric bushing (14) within said u-shaped saddle (13), said elastomeric bushings (14) comprising said isolation means (14).

24. The hand-held massager (1) of Claim 19, wherein said massage head (30) includes textured protuberances (32) on the surface thereof.

25. The hand-held massager (1) of Claim 19, wherein said vibration means (20) is a solenoid (28) energized by a repetitive signal.

26. The hand-held massager (1) of Claim 19, wherein said vibration means (20) is an electric motor (22) including at least one rotating eccentric weight (24).

27. The hand-held massager (1) of Claim 19, wherein said massage head (30) is substantially spherical.

28. The hand-held massager (1) of Claim 19, wherein said massage action is primarily non-parallel to said longitudinal axis (A2) of said handle (10).

29. The hand-held massager (1) of Claim 23, wherein each said elastomeric bushing (14) has approximately equal degrees of axial flex and radial flex.

30. The hand-held massager (1) of Claim 23, wherein each said elastomeric bushing (14) is substantially tubular and includes a support means (40) to prevent said elastomeric bushing (14) from collapsing on itself.

31. The hand-held massager (1) of Claim 23, wherein said massager includes means (16b, 50) for substantially preventing said axle means (16) from rotating when said axle means (16) ends are positioned within said elastomeric bushings (14).

32. The hand-held massager (1) of Claim 31 wherein said means (16b, 50) for preventing said axle means (16) from rotating include key means (16b, 50) on said axle means (16) ends and in said elastomeric bushings (14).

33. The hand-held massager (1) of Claim 19, wherein said massager (1) has a natural vibration frequency and said vibration means (20) operates at a vibration frequency of approximately 150% of said natural vibration frequency of said massager (1).

34. The hand-held massager (1) of Claim 19, wherein said massage head (30) contains air vents (34).

35. The hand-held massager (1) of Claim 19, wherein said vibration means (20) has a net center of gravity, and said vibration means (20) net center of gravity is located at a point approximately along said axis of rotation (A3) at the midpoint thereof.

36. The hand-held massager (1) of Claim 34, wherein said vibration means (20) further comprises an integral fan 23.

37. The hand-held massager (1) of Claim 33, further including means for automatically and periodically varying the vibration frequency of said vibration means (20).

38. The hand-held massager (1) of Claim 19, further comprising a power supply (67) selected from the group consisting of switching power supplies and capacitive potential divider power supplies.

39. The hand-held massager (1) of Claim 23, wherein said elastomeric bushings (14) comprise a material selected from the group consisting of polyurethane, natural, and synthetic rubbers.

40. The hand-held massager (1) of Claim 19, wherein said massager (1) contains a heating means (60).

41. The hand-held massager (1) of Claim 40, wherein said heating means (60) is selected from the group consisting of a resistive element, positive temperature coefficient elements, and infrared elements.

42. The hand-held massager (1) of Claim 19, wherein said massager (1) is powered by a rechargeable, self-contained power source (63).

43. The hand-held massager (1) of Claim 19, wherein said massager (1) is powered by a non-rechargeable, self-contained power source (63).

44. The hand-held massager (1) of Claim 19, wherein said massager (1) is powered by an external power source (65).

45. The hand-held massager (1) of Claim 19, including means for preventing said massage head (30) from rotating.

46. The hand-held massager (1) of Claim 19, wherein said massage action is substantially restricted to a single axis of motion.

47. The hand-held massager (1) of Claim 19, wherein said massage head (30) is covered with an elastomeric material.

48. The hand-held massager (1) of claim 19 further including means for varying vibration of said vibration means (20).

49. The hand-held massager (1) of Claim 40, wherein said heating means (60) is positioned within said massage head (30).

50. The hand-held massager (1) of Claim 49, wherein said heating means comprises said vibration means (20).
